# EUROPEAN PATENT APPLICATION

(11) **EP 3 222 240 A1**
(43) Date of publication of application: **27.09.2017**
(21) Application number: 15860661.6
(22) Date of filing: 05.10.2015
(51) Int. Cl.: A61B 18/14, A61B 17/221

(54) **HIGH FREQUENCY TREATMENT TOOL AND HIGH FREQUENCY TREATMENT SYSTEM**

(30) Priority: 21.11.2014 JP 2014236933
(71) Applicant: Olympus Corporation, Hachioji-shi, Tokyo 192-8507 (JP)
(72) Inventor: MOTOSUGI, Shunsuke, Hachioji-shi, Tokyo 192-8507 (JP); SATO, Yukio, Hachioji-shi, Tokyo 192-8507 (JP); ONUKI, Yoshio, Hachioji-shi, Tokyo 192-8507 (JP)
(74) Representative: Gunzelmann, Rainer
(86) International application number: PCT/JP2015/078192
(87) International publication number: WO 2016/080093

(57) **Abstract**

Provided is a high-frequency treatment tool. A positioning member (14) that regulates movement of a second wire part (16e) within a sheath (12) relative to a first wire part (16b) in a direction of a longitudinal axis of the second wire part (16e) is present in the sheath (12). In a state in which a folded part (16d) is located close to a distal end of the sheath (12), a loop formation part (16c) is fed from the distal end of the sheath (12) with the second wire part (16e) fixed.

## Description

### [Technical Field]

The present invention relates to a high-frequency treatment tool and a high-frequency treatment system.

Priority is claimed on Japanese Patent Application No. 2014-236933, filed on November 21, 2014, the content of which is incorporated herein by reference.

### [Background Art]

Recently, endoscopic mucosal resection (EMR) has attract attention as means for treating early membrane cancer. As an improvement to EMR, a method of suctioning a lesion portion into a transparent cap mounted on a tip of an endoscope, and resecting the lesion portion using a high-frequency snare formed at the end of the transparent cap (EMR using a cap (EMRC)) is known.

For example, living body tissue resection devices used to perform a procedure of EMRC are disclosed in Patent Documents 1 and 2.

### [Citation List]

### [Patent Document]

### [Patent Document 1]

Japanese Unexamined Patent Application, First Publication No. 2005-27722

### [Patent Document 2]

Japanese Unexamined Patent Application, First Publication No. 2008-73317

### [Summary of Invention]

### [Technical Problem]

In the procedure of EMRC using the device disclosed in Patent Document 1, a lesion portion suctioned into a transparent cap is a target to be resected, and an incising medical device such as a high-frequency snare is disposed for enclosing this lesion portion.

In the treatment tool disclosed in Patent Document 2, a snare wire is protruded from a sheath and then provided along the cap, and a loop large enough to surround a target to be resected is formed in the cap in advance.

When the snare wire is developed in the cap, it is necessary to dispose the snare wire in a proper loop shape at a proper scheduled position of incision while both an amount of protrusion of the snare wire from a treatment tool channel of the endoscope and a developed state of a loop-like portion of the snare wire are each adjusted. To be specific, since the loop portion of the snare wire that has already protruded from a tip of the sheath needs to be developed in the cap in a circular shape, work for simultaneously adjusting both an amount of protrusion of the loop-like portion from the tip of the sheath and a position of the tip of the sheath takes place. For this reason, the conventional EMRC requires skill.

The present invention has been made in view of the above circumstances, and it is an object of the present invention to provide a high-frequency treatment tool in which a procedure of EMRC is easier over the related art.

### [Solution to Problem]

An aspect of the present invention provides a high-frequency treatment tool used along with a cap that is attached to a distal end of an endoscope and the cap has a tubular inner circumferential surface. The high-frequency treatment tool includes: a tubular sheath having flexibility; a first wire part disposed along a longitudinal axis of the sheath; a loop formation part that is a wire continuous to a distal portion of the first wire part and enables elastic deformation to be bent in an annular shape when protruding from the distal portion of the sheath; a second wire part disposed along the longitudinal axis of the sheath; a folded part formed by folding a wire, which is configured to be projected from and retracted into a tip of the sheath and is continuous to a distal portion of the second wire part, in a U shape; a connection part disposed between a distal portion of the loop formation part and a proximal portion of the folded part, configured to bend the proximal portion of the folded part in a direction apart from the distal portion of the second wire part, and connected to the distal portion of the loop formation part; an operation part provided at a proximal portion of the first wire part and configured to enable manipulation such that the folded part, the loop formation part, and the connection part protrude from the tip of the sheath; a stopper provided at a proximal portion of the second wire part and configured to have a diameter larger than an outer diameter of the second wire part; and a positioning member having a contact surface set to come into contact with the stopper at a position at which an amount of protrusion at the loop formation part from the sheath along an axis of the loop formation part is shorter than a circumferential length of an inner circumferential surface at a distal end of the cap.

The folded part and the connection part may protrude from the tip of the sheath in a state in which the stopper is in contact with the contact surface of the positioning member.

The high-frequency treatment tool of the aspect may further include a second connection part configured to bend the proximal portion of the folded part in a direction apart from the distal end of the first wire part between the distal portion of the second wire part and the proximal portion of the folded part and connect the distal portion of the second wire part and the proximal portion of the folded part.

An interval between the wires of the folded part may be smaller than an inner diameter of the sheath.

The stopper may include a fixing part to which a proximal end of the second wire part is fixed, and a passage part into which the first wire part is inserted to move forward and backward. A distal end of the stopper and a proximal end of the positioning member may come into contact with each other, and thereby the positioning member may regulate movement of the second wire part. When a state in which the stopper and the positioning member are spaced from each other is changed to a state in which the stopper and the positioning member are in contact with each other, the outer sheath is capable of moving with respect to the inner sheath such that a distal end of the outer sheath is capable of being disposed close to the folded part.

The sheath may include an inner sheath in which the positioning member is fixed, and an outer sheath into which the inner sheath is inserted. In a state in which the positioning member regulate movement of the stopper, a distal end of the outer sheath may allow of movement relative to the inner sheath to be able to be disposed close to the folded part.

The positioning member may include a first passage part into which the first wire part is inserted, and a second passage part into which the second wire part is inserted, and the positioning member may have conductivity and allow a high-frequency current to be carried.

The sheath may have a marking at a position entering a visual field of the endoscope at a timing at which the folded part is located at a distal end portion of the cap.

Another aspect of the present invention provides a high-frequency treatment system that includes: a cap attached to a distal end of an endoscope and having a tubular inner circumferential surface; and a high-frequency treatment tool inserted into a channel of the endoscope. The high-frequency treatment tool includes: a tubular sheath having flexibility; a first wire part disposed along a longitudinal axis of the sheath; a loop formation part that is a wire continuous to a distal portion of the first wire part and enables elastic deformation to be bent in an annular shape when protruding from the distal portion of the sheath; a second wire part disposed along the longitudinal axis of the sheath; a folded part formed by folding a wire, which is configured to be projected from and retracted into a tip of the sheath and is continuous to a distal portion of the second wire part, in a U shape; a connection part disposed between a distal portion of the loop formation part and a proximal portion of the folded part, configured to bend the proximal portion of the folded part in a direction apart from the distal portion of the second wire part, and connected to the distal portion of the loop formation part; an operation part provided at a proximal portion of the first wire part and configured to enable manipulation such that the folded part, the loop formation part, and the connection part protrude from the tip of the sheath; a stopper provided at a proximal portion of the second wire part and configured to have a diameter larger than an outer diameter of the second wire part; and a positioning member having a contact surface set to come into contact with the stopper at a position at which an amount of protrusion of the loop formation part from the sheath along an axis of the loop formation part is shorter than a circumferential length of an inner circumferential surface at a distal end of the cap. The cap has a loop holding surface that protrudes from the inner circumferential surface toward a central line of the cap and with which the folded part allows of contact.

### [Advantageous Effects of Invention]

According to the present invention, a procedure of EMRC is easier over the related art.

### [Brief Description of Drawings]

Fig. 1 is an overall view showing a state in which an endoscopic tissue resection device of a first embodiment of the present invention is attached to an endoscope.
Fig. 2 is a partial cross-sectional view showing a resection tool of the endoscopic tissue resection device.
Fig. 3 is a view for showing an operation of the resection tool.
Fig. 4 is a view for showing an operation of the endoscopic tissue resection device.
Fig. 5 is a view for showing an operation of the endoscopic tissue resection device.
Fig. 6 is a view for showing an operation of the endoscopic tissue resection device.
Fig. 7 is a schematic view for showing a procedure of EMRC using the endoscopic tissue resection device.
Fig. 8 is a view showing a constitution of a modification of the first embodiment, and schematically showing an endoscope image when an endoscopic tissue resection device of the modification is used.
Fig. 9 is a cross-sectional view showing an endoscopic tissue resection device of a second embodiment of the present invention.
Fig. 10 is a view for showing an operation of the endoscopic tissue resection device.
Fig. 11 is a cross-sectional view showing an endoscopic tissue resection device of a third embodiment of the present invention.
Fig. 12 is a side view showing another constitution example of the endoscopic tissue resection device of the third embodiment.

### [Description of Embodiments]

### (First embodiment)

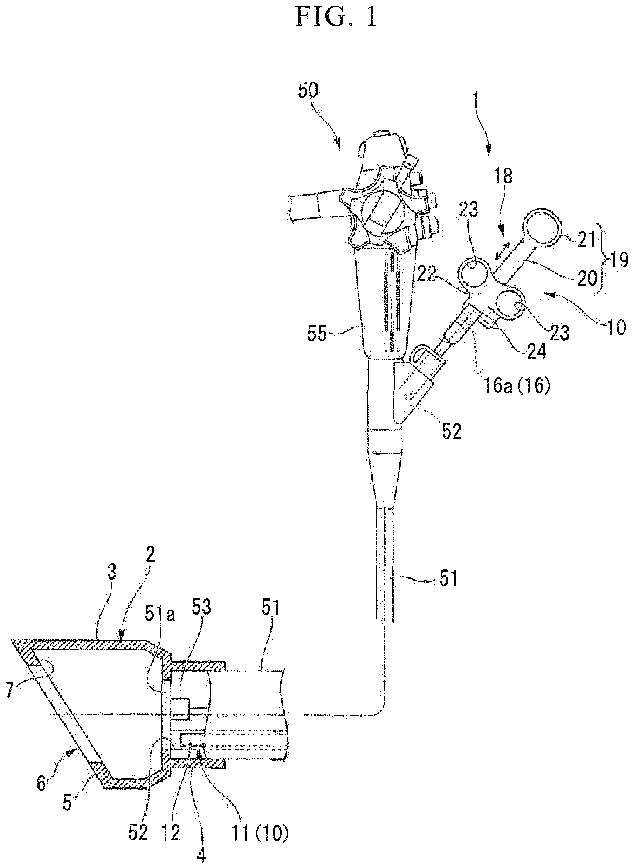

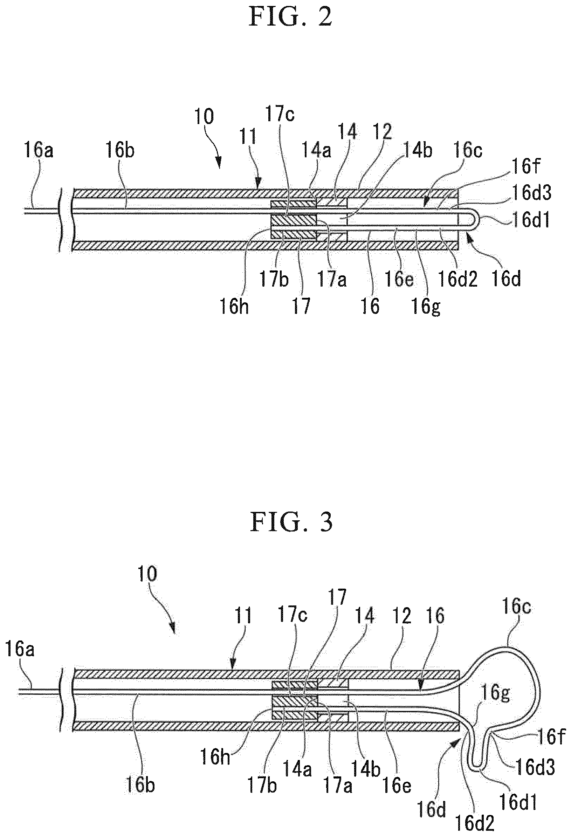

An endoscopic tissue resection device (a high-frequency treatment system) 1 (hereinafter referred to simply as "tissue resection device 1") of a first embodiment of the present invention will be described. Fig. 1 is an overall view showing a state in which the endoscopic tissue resection device of the present embodiment is attached to an endoscope. Fig. 2 is a partial cross-sectional view showing a resection tool of the endoscopic tissue resection device. Fig. 3 is a view for showing an action of the resection tool.

As illustrated in Fig. 1, the tissue resection device 1 is a medical device has a cap 2 and a resection tool (a high-frequency treatment tool) 10 and can be used with an endoscope 50. The tissue resection device 1 of the present embodiment may be used after it is attached to the known endoscope 50. The known endoscope 50 has, for instance, an insertion body 51 and an operation body 55. The insertion body 51 has a treatment tool channel 52 for guiding the resection tool 10 up to a treatment target region. An image pickup unit 53 for observing the treatment target region is disposed at a distal end of the insertion body 51.

The cap 2 is a member that can be attached to a distal end 51 a of the endoscope 50. At least a part of the cap 2 is preferably transparent at a portion at which it overlaps a visual field of the endoscope 50 such that it does not obstruct the visual field of the endoscope 50. The entire cap 2 is preferably formed of a transparent material.

The cap 2 has an approximately tubular shape, and has a tube part 3 having openings in opposite ends thereof, and a loop holder 6 disposed at one of the openings of the tube part 3. In the present embodiment, of the two openings formed in the tube part 3, a side (an proximal end side of the cap 2) at which the open end runs along a plane orthogonal to a central line of the cap 2 is an endoscope coupler part 4 that can be attached to the insertion body 51 of the endoscope 50, and a side (a distal end side of the cap 2) at which the open end runs along a plane inclined with respect to the central line of the cap 2 is a contact part 5 that can be brought into contact with the treatment target region (the resection target region). An oblique angle of the contact part 5 is set on the basis of an approach angle of the endoscope 50 to the treatment target region.

The endoscope coupler part 4 is provided for the tube part 3 to be able to be coupled to the endoscope 50 such that the image pickup unit 53 of the endoscope 50 is disposed inside the tube part 3. For example, a distal end of the insertion body 51 can be inserted into and fixed to the endoscope coupler part 4.

The loop holder 6 is an annular portion that is disposed close to the contact part 5 out of the cap 2. The loop holder 6 has a distal end of an inner circumferential surface of the tube part 3, and an annular portion that is continuous to the distal end of the inner circumferential surface of the tube part 3 and protrudes from the inner circumferential surface of the tube part 3 toward the central line of the cap 2. The loop holder 6 has a loop holding surface 7. The loop holding surface 7 is a surface that is approximately directed to the endoscope coupler part 4 side, and can come into contact with a wire of the resection tool 10. The loop holding surface 7 of the loop holder 6 of the present embodiment regulates a shape and a size of the opening of the distal portion of the cap 2 when the cap 2 is attached to the endoscope 50.

The resection tool 10 illustrated in Figs. 1 and 2 is a high-frequency treatment tool that can resect a region to be treated in tissue of a living body. The resection tool 10 is used with the cap 2 attached to the distal end of the endoscope 50. The resection tool 10 has an insertion part 11 that is inserted into the body, and an operation part 18 that is disposed at a proximal end of the insertion part 11.

As illustrated in Fig. 2, the insertion part 11 has a sheath 12 that can be inserted into the treatment tool channel 52 of the endoscope 50, a positioning member 14 that is fixed to the sheath 12, a wire (a snare wire) 16 that is disposed inside the sheath 12, and a stopper 17 that is disposed inside the sheath 12.

The sheath 12 is an approximately tubular member that has flexibility. The sheath 12 is a tube formed of, for instance, a resin.

The positioning member 14 is in a shape that protrudes from an inner surface of the sheath 12 to an inner side of the sheath 12. The positioning member 14 of the present embodiment is a tubular member that has a proximal end face 14a with which a distal end face 17a of the stopper 17 (to be described below) can come into contact and a through-hole part 14b that has an inner diameter to such a degree that the wire 16 (to be described below) moves freely forward and backward and extends in a direction of the central line of the sheath 12. The positioning member 14 may not be in tubular shape.

The wire 16 is a member of a wire shape that is disposed in the sheath 12 to be projectable and retractable from the distal end of the sheath 12 and is bent in a predetermined shape to come into contact with tissue to be resected and resect the tissue.

The wire 16 of the present embodiment can be projected from and retracted into the distal end of the sheath 12 in response to manipulation caused by the operation part 18 (to be described below) (see Fig. 1).

The wire 16 is a conductive member to which a high-frequency current is carried from a high-frequency power supply (not shown). The wire 16 can incise tissue when the wire 16 comes into contact with the tissue in a state in which the high-frequency current is carried to the wire 16. A cross-sectional shape of the wire 16 is not particularly restricted. For example, the wire 16 of the present embodiment is a conductive member whose cross section is in a circular shape.

The wire 16 has a first wire part 16b, a loop formation part 16c, a folded part 16d, a second wire part 16e, a first connection part 16f, and a second connection part 16g between opposite ends (first and second ends 16a and 16h) thereof.

The first end 16a of the wire 16 is fixed to a slider part 22 of the operation part 18. Further, the first end 16a of the wire 16 is electrically connected to a connector 24 (to be described below) disposed at the slider part 22. A high-frequency current is carried to the wire 16 via the connector 24.

The first wire part 16b is a wire-like portion that extends from the slider part 22 of the operation part 18 toward a distal side thereof through the inside of the sheath 12 in the entire wire 16. The first wire part 16b extends along a longitudinal axis of the sheath 12. A distal portion of the first wire part 16b is continuous to a proximal portion of the loop formation part 16c. The first wire part 16b is inserted into the stopper 17 in the sheath 12 to move freely forward and backward relative to the stopper 17 (to be described below).

The loop formation part 16c is a wire-like portion that is set between a distal end face 16d1 of the folded part 16d and the first end 16a of the wire 16 out of the entire wire 16 having a U shape. The loop formation part 16c is soft enough to be deformed along an inner surface of the cap 2 (see Fig. 1). A shape of the loop formation part 16c is set to easily have a loop shape (see Fig. 3) surrounding a resection target region in the cap 2. For example, the loop formation part 16c may be formed in a curved shape in a state in which an external force is not applied, and thereby form a predetermined curved shape outside of the sheath 12 to follow the inner surface of the cap 2. By way of example, the first connection part 16f is folded in a direction in which the loop formation part 16c is apart from the first proximal portion 16d2 of the folded part 16d. For this reason, the loop formation part 16c is bent in a loop shape such that an interval between wires is greater than an outer diameter of the sheath 12 in a state in which the loop formation part 16c protrudes from the sheath 12.

When a distal end face 17a of the stopper 17 comes into contact with a proximal end face 14a of the positioning member 14, a length running along an axis of the loop formation part 16c in the loop formation part 16c protruding from the sheath 12 is shorter than a circumferential length of an inner circumferential surface at the distal end of the cap 2 (a circumferential length of a portion at which an outermost diameter of the loop holding surface 7 is great). In a contact state in which the distal end face 17a of the stopper 17 comes into contact with the proximal end face 14a of the positioning member 14, only the loop formation part 16c is unreeled from the sheath 12. For this reason, in this contact state, when the distal end face 16d1 of the folded part 16d comes into contact with the loop holding surface 7 of the cap 2, the wire can be made to run along the loop holding surface 7 while the loop formation part 16c is being unreeled from the sheath 12. According to this, since there is no trouble of developing the wire 16, which has already protruded from the sheath 12, inside of the cap in an annular shape as in the related art, work for simultaneously adjusting both an amount of protrusion of the wire 16 protruding from the sheath 12 and a position of the sheath 12 is not also required.

In the present embodiment, the loop formation part 16c is developed inside the cap 2 at a boundary portion between the loop holding surface 7 (see Fig. 1) formed at the cap 2 and the inner surface of the cap 2 to follow an oval shape formed by this boundary portion.

The folded part 16d is a portion which is formed in a shape folded about 180° in a U shape out of the entire wire 16, and at which a curved portion and wires (the first and second proximal portions 16d2 and 16d3) continuous to the curved portion extend in an approximately linear shape. A size of the folded part 16d has such a size that the folded part 16d can be housed inside the sheath 12. That is, the folded part 16d is folded in the U shape such that an interval between the wires (an interval between the aforementioned first and second proximal portions 16d2 and 16d3) in the state in which the folded part 16d protrudes from the sheath 12 is smaller than an inner diameter of the sheath 12. When the interval between the first proximal portion 16d2 and the second proximal portion 16d3 is smaller than the inner diameter of the sheath 12, an operator easily checks an amount of protrusion of the folded part 16d on an endoscope image. The interval between the aforementioned wires is not necessarily required to be smaller than the inner diameter of the sheath 12. The distal end face 16d1 of the folded part 16d is pressed against the loop holding surface 7 that is the distal end at the cap 2.

In the present embodiment, to regulate the loop shape of the loop formation part 16c, a joint (the first connection part 16f) between the second proximal portion 16d3 of the folded part 16d and the loop formation part 16c is bent in a direction in which it is apart from the first proximal portion 16d2. A joint (the second connection part 16g) between the first proximal portion 16d2 of the folded part 16d and a distal portion of the second wire part 16e is also bent in a direction in which it is apart from the second proximal portion 16d3. To be specific, in the state in which the folded part 16d is housed in the sheath 12, each of the first connection part 16f and the second connection part 16g has a bending inclination such that it is directed to the outside of the sheath 12 in a radial direction. The bending inclination given to the second connection part 16g makes it easy for the loop formation part 16c to be developed inside the cap 2 in an annular shape. The bending inclination at the second connection part 16g is not essential.

The second wire part 16e is a wire-like portion that is disposed in the sheath 12 at a shorter length than the first wire part 16b to regulate the amount of protrusion of the folded part 16d from the sheath 12. The distal portion of the second wire part 16e is continuous to the first proximal portion 16d2 of the folded part 16d (to be described below). A proximal portion of the second wire part 16e includes the second end 16h of the wire 16. The stopper 17 is fixed to the second end 16h of the wire 16. Movement of the stopper 17 toward the distal side is restricted by the positioning member 14.

The stopper 17 illustrated in Fig. 2 is attached to the wire 16 such that a part of the wire 16 is restricted in movement toward the distal side by the positioning member 14.

The stopper 17 has a distal end face 17a that can come into contact with the proximal end face 14a of the positioning member 14, a wire fixing part 17b to which the second end 16h of the wire 16 is fixed, and a wire passage part 17c into which the first wire part 16b serving as an intermediate portion of the wire 16 is inserted.

The stopper 17 is a tubular member in which two through-holes, central lines of which extend in parallel to each other, are formed. One of the two through-holes is the wire fixing part 17b, and the other through-hole is the wire passage part 17c.

The wire fixing part 17b is a portion at which the second end 16h of the wire 16 is inserted from a distal side toward a proximal side of the stopper 17 and is fixed by a known fixing method. A method of fixing the wire 16 to the wire fixing part 17b is, for instance, brazing or welding.

The wire passage part 17c is a portion in which a through-hole having such an inner diameter that the wire 16 can move forward and backward therein is formed. A cross-sectional shape of the wire passage part 17c which is orthogonal to a central line thereof follows a contour shape of the wire 16 in a cross section orthogonal to a central line of the wire 16.

The operation part 18 illustrated in Fig. 1 has a main body part 19 fixed to a proximal end of the sheath 12, and a slider part 22 attached to the main body part 19.

The main body part 19 has a shaft part 20 having a central line on the same axis as the sheath 12, and a finger hooking ring 21 formed at a proximal end of the shaft part 20.

The shaft part 20 is a member that has an approximate rod shape in which a distal end thereof is fixed to the proximal end of the sheath 12. A space in which the wire 16 is disposed is provided inside the shaft part 20. The shaft part 20 guides the slider part 22 (to be described below) to displace the slider part 22 along a central line of the shaft part 20.

The finger hooking ring 21 is a ring having an inner diameter large enough for an operator to insert a finger.

The operator inserts his/her finger into the finger hooking ring 21, and thereby can stably hold the shaft part 20.

The slider part 22 is a member that projects and retracts the wire 16 from the sheath 12 to thereby develop the loop formation part 16c or store the loop formation part 16c in the sheath 12. The slider part 22 is a member that can move relative to the shaft part 20 in a direction of the central line of the shaft part 20, and has finger hooking parts 23 and a connector 24. In the present embodiment, when the slider part 22 moves relative to the shaft part 20 in the direction of the central line of the shaft part 20, the slider part 22 displaces the first end 16a of the wire 16 in the direction of the central line of the shaft part 20.

The finger hooking parts 23 has a plurality of rings, each of which has an inner diameter large enough for an operator to insert a finger. The operator inserts his/her fingers into the respective rings of the finger hooking parts 23, and thereby can stably hold the slider part 22.

The operator passes his/her fingers through the finger hooking ring 21 of the shaft part 20 and the finger hooking parts 23 of the slider part 22, respectively. Thereby, the operator easily enables the slider part 22 to move forward or backward relative to the shaft part 20 along the central line of the shaft part 20 in a proximal or distal direction.

The connector 24 is a conductor that is fixed to the slider part 22 to carry a high-frequency power source to the wire 16 from a high-frequency power supply (not shown). The first end 16a of the wire 16 is fixed to the connector 24.

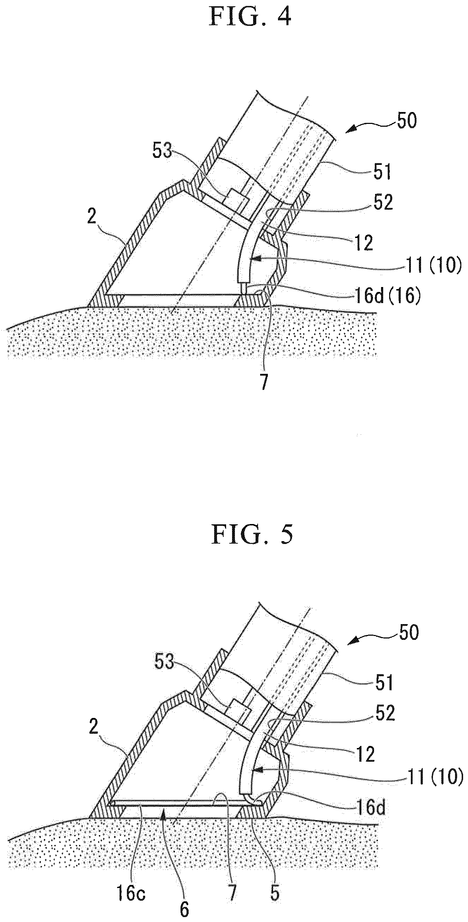

Next, an operation of the tissue resection device 1 of the present embodiment will be described. Figs. 4 to 6 are views showing the operation of the endoscopic tissue resection device 1 of the present embodiment.

Hereinafter, an operation of the tissue resection tool 10 of the present embodiment will be described by giving a procedure of an endoscopic mucosal resection (EMR) using a cap (EMRC) that resects mucosal tissue by using a combination of the endoscope 50, the resection tool 10, and the cap 2 by way of example.

When the tissue resection device 1 is used, the cap 2 is attached to the distal end of the insertion body 51 of the endoscope 50 first, as illustrated in Fig. 1. Subsequently, the endoscope 50 is guided to tissue to be resected by a known procedure. Prior to starting to use the tissue resection device 1, a counter electrode plate (not shown) connected to a high-frequency power supply as a counter electrode for the loop formation part 16c of the resection tool 10 is attached to the body of a patient.

A user of the tissue resection device 1 brings the distal end of the cap 2 into contact with the tissue to be resected (see Fig. 4), and adjusts a position of the cap 2 such that a resection target region is fitted into an opening located at the distal portion of the cap 2.

Subsequently, the user of the tissue resection device 1 guides the insertion part 11 of the resection tool 10 to the resection target region through the treatment tool channel 52 of the endoscope 50, as illustrated in Fig. 4. The wire 16 is pulled in in a proximal direction of the sheath 12 to such a degree that the folded part 16d of the wire 16 is located inside the sheath 12 until the insertion part 11 of the resection tool 10 is inserted into the treatment tool channel 52 and protrudes from the distal end of the treatment tool channel 52.

A portion of the entire resection tool 10 which protrudes from the distal end of the treatment tool channel 52 of the endoscope 50 is partly located at a position that can be observed by an operator using an endoscope image. The operator guides the distal end of the sheath 12 to a predetermined position in the cap 2 with reference to the endoscope image. The sheath 12 can be positioned in the cap 2 by advancing/retreating or rotating the entire operation part relative to the treatment tool channel 52. The predetermined position of the distal end of the sheath 12 in the cap 2 is a position at which the folded part 16d can come into contact with the loop holding surface 7 when the wire 16 is displaced along the central line of the sheath 12 in the distal direction of the sheath 12.

In the present embodiment, the position of the distal end of the sheath 12 regulates a resection position for the resection target region. That is, a side at which the position of the distal end of the sheath 12 is adjacent to the loop holding surface 7 can more widely resect the resection target region in the cap 2.

A gap required to develop the loop formation part 16c is present between the distal end of the sheath 12 and the loop holding surface 7 of the cap 2. A size of the gap between the distal end of the sheath 12 and the loop holding surface 7 of the cap 2 may be adequately adjusted by an operator on the basis of the endoscope image. The sheath 12 is held in the cap 2 by the treatment tool channel 52 to take a posture in which the wire 16 can extend toward the loop holding surface 7 when the wire 16 protrudes from the distal end of the sheath 12.

Subsequently, as the slider part 22 illustrated in Fig. 1 is displaced along the central line of the shaft part 20 in the distal direction of the shaft part 20 by the operator, the first end 16a of the wire 16 moves in the distal direction. Thereby, the wire 16 moves along the central line of the sheath 12 from the proximal end toward the distal end of the sheath 12.

As illustrated in Fig. 2, the folded part 16d of the wire 16 is pushed in the distal direction by an amount of force transmitted from the first end 16a (see Fig. 1). The second end 16h of the wire 16 is pulled by the folded part 16d and moves in the distal direction of the sheath 12. For this reason, the entire wire 16 moves along the central line of the sheath 12 from the proximal direction to the distal direction of the sheath 12 until the stopper 17 comes into contact with the positioning member 14.

When the distal end face 17a of the stopper 17 comes into contact with the proximal end face 14a of the positioning member 14, the second end 16h is regulated to be unable to further move in the distal direction in a direction of a longitudinal axis of the sheath 12. At this point, as illustrated in Fig. 4, the folded part 16d is located at a position that is adjacent to the loop holding surface 7 or at a position at which the distal end face 16d1 of the folded part 16d is in contact with the loop holding surface 7. Further, in a state in which the distal end face 17a of the stopper 17 is in contact with the proximal end face 14a of the positioning member 14, the folded part 16d, the loop formation part 16c, and the first connection part 16f protrude from a tip of the sheath 12. Further, in the state in which the distal end face 17a of the stopper 17 is in contact with the proximal end face 14a of the positioning member 14, the amount of protrusion of the loop formation part 16c from the sheath 12 (the length running along the axis of the wire 16 at the loop formation part 16c) is smaller than the circumferential length of the inner circumferential surface of the distal end of the cap 2 (i.e. the circumferential length of the portion at which the outer diameter of the loop holding surface 7 is greatest).

When the first end 16a is further displaced in the distal direction by the slider part 22 after the distal end face 17a of the stopper 17 comes into contact with the proximal end face 14a of the positioning member 14, the first wire part 16b of the wire 16 moves through the inside of the wire passage part 17c of the stopper 17 in the distal direction of the sheath 12. For this reason, a region of the entire wire 16 which is closer to the first end 16a than the folded part 16d is pushed against a region of the entire wire 16 which is closer to the second end 16h than the folded part 16d in the distal direction. For this reason, the region of the entire wire 16 which is closer to the first end 16a than the folded part 16d has, as illustrated in Fig. 3, an approximately arcuate shape at a portion exposed to the outside of the sheath 12. That is, the development of the loop formation part 16c is initiated by a force with which the wire 16 is further pushed out of the state in which the stopper 17 is in contact with the positioning member 14. In the present embodiment, since the loop formation part 16c protrudes from the sheath 12 and thereby becomes a loop shape in the state in which the second end 16h of the wire 16 is fixed, the development of the loop formation part 16c is possible although the wire 16 is not supported by the cap 2. Further, in a process of developing the loop formation part 16c, the wire 16 comes into contact with the loop holding surface 7. Afterwards, with the development of the loop formation part 16c, the loop formation part 16c is held on the loop holding surface 7 in a circumferential direction of the inner circumferential surface of the distal end of the cap 2.

In the conventional snare, it is necessary to adjust a position of the sheath into which the snare is inserted to form a loop at the tip portion of the cap. However, in the present embodiment, it is unnecessary to adjust the position of the sheath 12 in the process of developing the loop formation part 16c.

Moreover, in the conventional snare, it is necessary to apply a force for pushing out the snare using support caused by the cap and, at this point, the snare is easily detached from the cap. However, in the present embodiment, the wire 16 is hardly detached from the cap 2.

As the first end 16a of the wire 16 is pushed out of the opening of the distal end of the sheath 12 by the slider part 22, the developed loop formation part 16c is, as illustrated in Fig. 5, disposed on the loop holding surface 7 according to the annular shape of the loop holding surface 7. Thereby, the loop formation part 16c is formed in an annular shape that follows the inner surface of the cap 2, and is held by the loop holder 6.

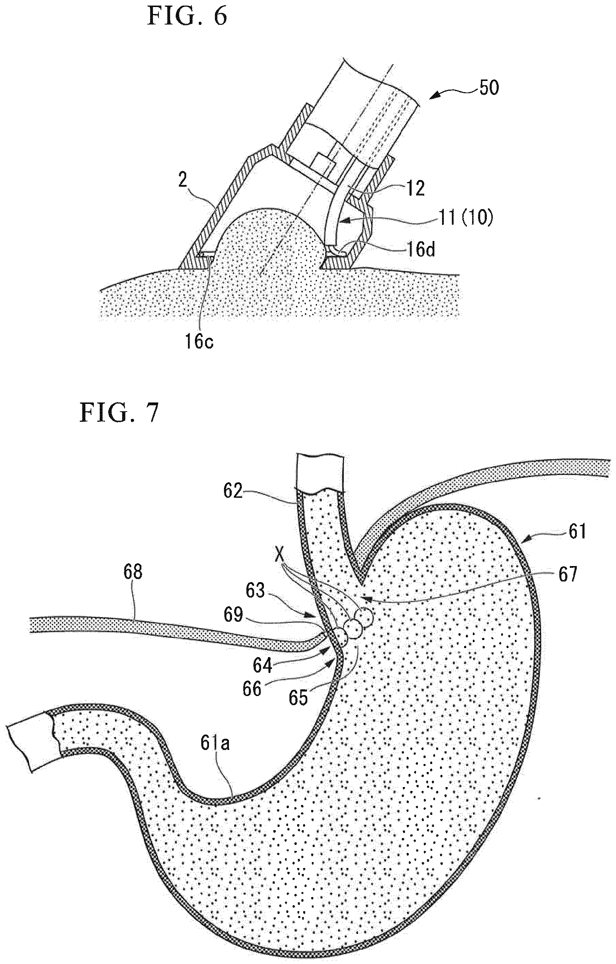

Subsequently, an operator forms negative pressure inside the cap 2 using a suction means (not shown) provided for the endoscope 50. Thereby, as illustrated in Fig. 6, the resection target region is pulled into the cap 2. In the cap 2, as the resection target region is pulled into the cap 2 from the opening of the distal portion of the cap 2, the resection target region enters a region in which the loop formation part 16c comes into contact with the loop holding surface 7 to become the annular shape.

In a state in which the resection target region is pulled into the cap 2, the operator displaces the slider part 22 illustrated in Fig. 1 along the central line of the shaft part 20 in the proximal direction of the shaft part 20. Then, the first end 16a fixed to the slider part 22 is displaced in the proximal direction by the slider part 22. As the first end 16a is displaced in the proximal direction by the slider part 22, the annular region formed by the loop formation part 16c as illustrated in Fig. 6 is reduced, and comes into contact with the resection target region. The resection target region is enclosed by the loop formation part 16c, and the loop formation part 16c comes into contact with the resection target region. In this state, as the high-frequency current is carried to the loop formation part 16c from the high-frequency power supply (not shown), the resection target region is incised. When the slider part 22 is displaced to the proximal side with the high-frequency current carried to the loop formation part 16c, the resection target region is completely excised by the loop formation part 16c.

After the resection target region is excised, the resection target region may be extracted from the body as needed.

Moreover, after the resection target region is excised, the tissue resection device 1 of the present embodiment and the endoscope 50 are extracted from the body.

In the conventional EMRC method, when the wire is developed to enclose the resection target region, an amount of extrusion of the wire from the sheath and a position of the sheath need to be adjusted in parallel.

In the conventional instrument used in the EMRC method, a procedure for the wire being formed in an annular shape to enclose the resection target region at a proper position is complicated.

In the resection tool 10 of the tissue resection device 1 of the present embodiment, the loop formation part 16c is fed out of the distal end of the sheath 12 while the second wire part 16e is being fixed in a state in which the folded part 16d is completely exposed from the sheath 12 in the vicinity of the distal end of the sheath 12. For this reason, the loop formation part 16c is adapted to start to be developed just after the loop formation part 16c starts to protrude from the sheath 12. Since the loop formation part 16c is held along the loop holding surface 7 in the process of developing the loop formation part 16c, it is unnecessary to bring the loop formation part 16c into contact with the cap 2 for development of the loop formation part 16c, and the wire 16 hardly receive a force by which the wire 16 is bent from the cap 2. For this reason, forming the wire 16 in a proper loop shape is easier than the related art. For example, as the development of the loop formation part 16c is initiated after the position of the sheath 12 is previously determined, when the loop formation part 16c is extruded from the sheath 12, the loop formation part 16c is naturally developed along the loop holding surface 7 in a proper loop shape, and thus the position of the sheath 12 and the position of the wire 16 need not to be adjusted at the same time. For this reason, the tissue resection device 1 of the present embodiment is easily manipulated during the development of the loop formation part 16c, compared to the related art.

Next, an example of a concrete procedure using the tissue resection device 1 of the present embodiment will be described. Fig. 7 is a schematic view showing a procedure of EMRC using the tissue resection device of the present embodiment.

In the present embodiment, as illustrated in Fig. 7, a gastroesophageal reflux disease occurs due to lower esophageal sphincter relaxation in a state in which a position of a gullet stomach joint region 63 and a position of a gullet hiatus 69 nearly coincide with each other, resection of tissue caused by the procedure of the EMRC is performed multiple times in the vicinity of the gullet stomach joint region 63, and a cardiac region 64 is reconstructed by generating cicatricial stenosis at a resected region.

The example of the above procedure is merely an example, the tissue resection device 1 of the present embodiment can be applied to the EMRC procedure for any tissue.

### (Modification)

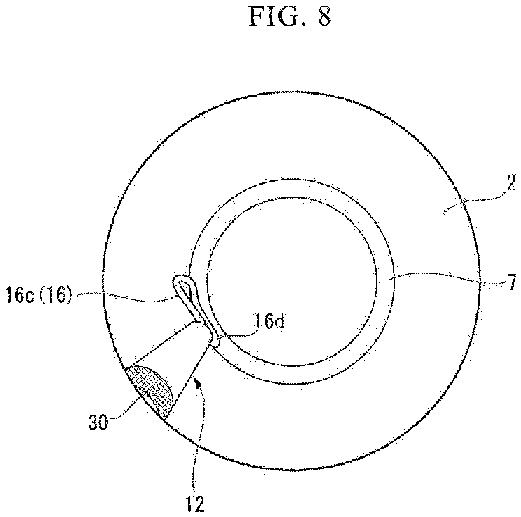

Next, a modification of the present embodiment will be described. Fig. 8 is a view showing a constitution of a modification of the present embodiment. Fig. 8 schematically illustrates an example of an endoscope image when an endoscopic tissue resection device of the present modification is used.

As illustrated in Fig. 8, in the present modification, the sheath 12 has a marking 30 that can be checked using an endoscope image.

The marking 30 is formed on an outer surface of the sheath 12, for instance, by printing or two-color formation.

A position of the marking 30 on the outer surface of the sheath 12 is set to be within a visual field of the image pickup unit 53(see Fig. 1) of the endoscope 50 at a timing at which a distance suitable for initiating the development of the loop formation part 16c is secured between the distal end of the sheath 12 and the loop holding surface 7.

The marking 30 is continuous on the outer surface of the sheath 12 in a circumferential direction. Alternatively, the marking 30 is disposed at multiple places of the outer surface of the sheath 12 to be able to be on the endoscope image when the sheath 12 is rotated about the central line of the sheath 12 in the treatment tool channel 52 illustrated in Fig. 1.

In the present modification, although it cannot be checked by the endoscope image whether the folded part 16d protrudes from the distal end of the sheath 12, the development of the loop formation part 16c is initiated on the basis of a position of the marking 30, and thereby, like the above embodiment, the loop formation part 16c can be developed in a proper shape in a state in which the wire 16 is in contact with the loop holding surface 7.

### (Second embodiment)

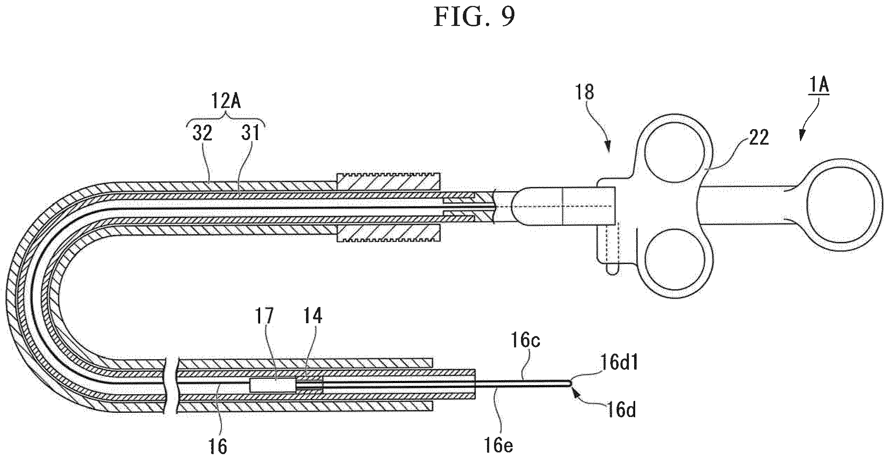

Next, a second embodiment of the present invention will be described. In each of the following embodiments, the same components as in the first embodiment are given reference signs shown in the first embodiment, and description repeated with that of the first embodiment will be omitted. Fig. 9 is a cross-sectional view showing an endoscopic tissue resection device of the present embodiment.

The endoscopic tissue resection device 1 A of the present embodiment is different in that, in place of the sheath 12 described in the above embodiment, it has a sheath 12A of a dual pipe structure due to an inner sheath 31 and an outer sheath 32.

The positioning member 14 described in the first embodiment is fixed to an inner surface of the inner sheath 31 in the present embodiment. A operation part 18 is fixed to a proximal end of the inner sheath 31.

The outer sheath 32 is attached to the inner sheath 31 to freely move forward and backward relative to the inner sheath 31. A distal end of the outer sheath 32 can be located at a farther distal side than a distal end of the inner sheath 31 by the forward/backward movement of the outer sheath 32 relative to the inner sheath 31.

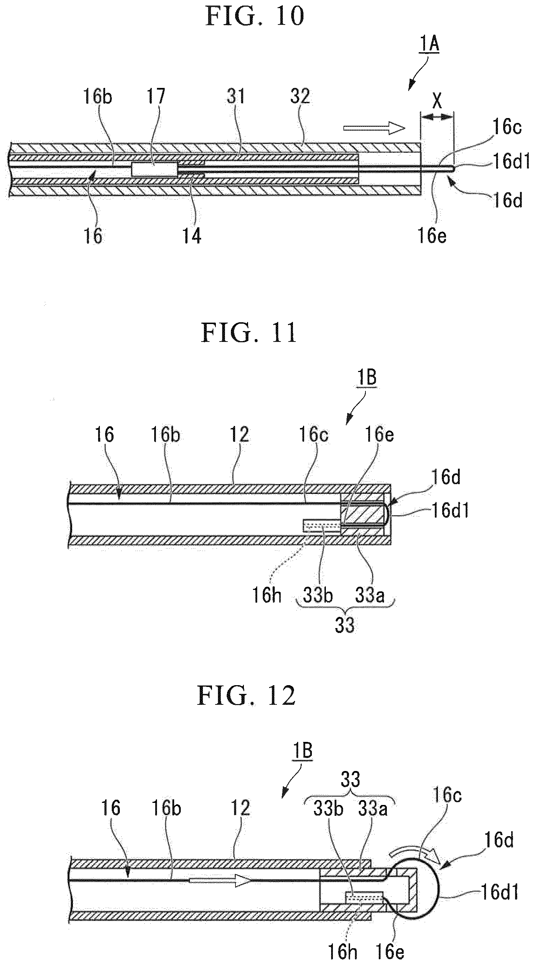

Next, an operation of the endoscopic tissue resection device 1A of the present embodiment will be described. Fig. 10 is a view showing an operation of the endoscopic tissue resection device 1A.

As illustrated in Figs. 9 and 10, an operator manipulates a slider part 22 of the operation part 18 like the first embodiment, and thereby the loop formation part 16c protrudes from the distal end of the inner sheath 31.

In the present embodiment, when the distal end of the outer sheath 32 is located at the proximal side relative to the distal end of the inner sheath 31, development of a loop formation part 16c is initiated at a point in time at which a folded part 16d protrudes from the distal end of the inner sheath 31.

In the present embodiment, when the distal end of the outer sheath 32 is located at the farther distal side than the distal end of the inner sheath 31, the development of the loop formation part 16c is initiated at a point in time at which the folded part 16d protrudes from the distal end of the outer sheath 32.

In this way, in the present embodiment, a position of the outer sheath 32 is adjusted to the inner sheath 31, and thereby a position at which the development of the loop formation part 16c is initiated can be changed. In the state in which the distal end of the outer sheath 32 is located at the farther distal side than the distal end of the inner sheath 31, the folded part 16d protrudes from the distal end of the outer sheath 32. In this state, a position of the inner sheath 31 is fixed, and the outer sheath 32 is pulled to a proximal side. Thereby, the loop formation part 16c can be developed without substantially changing a position of a distal end face 16d1 of the folded part 16d.

In the present embodiment, since a method of developing the loop formation part 16c has more choices than in the first embodiment, a possibility of selecting a development order corresponding to a situation of a resection target region to easily develop the loop formation part 16c is higher than in the first embodiment.

In the present embodiment, an outer sheath 32 may have the marking 30 disclosed in the modification of the first embodiment. In addition, the marking 30 disclosed in the modification of the first embodiment may be provided for both the outer sheath 32 and an inner sheath 31.

### (Third embodiment)

Next, a third embodiment of the present invention will be described. Fig. 11 is a cross-sectional view showing an endoscopic tissue resection device of the present embodiment. Fig. 12 is a side view showing another constitution example of the endoscopic tissue resection device of the present embodiment.

In the endoscopic tissue resection device 1B of the present embodiment illustrated in Fig. 11, the positioning member 14 and the stopper 17 described in the first embodiment are integrated, which is different from the first embodiment.

That is, in the present embodiment, in place of the positioning member 14 and the stopper 17, a stopper 33 becoming the same as the state in which the stopper 17 is in contact with the positioning member 14 in the first embodiment is fixed to the inside of a sheath 12.

A wire passage part 33a having through-holes into which a wire 16 is inserted to freely move forward and backward, and a wire fixing part 33b for fixing a second end 16h of the wire 16 are formed at the stopper 33. The stopper 33 is formed of a conductor.

In the present embodiment, as the slider part 22 of a operation part 18 is displaced like the first embodiment, the wire 16 is extruded from the wire passage part 33a, and development of a loop formation part 16c is initiated.

A direction of each through-hole in the wire passage part 33a formed at the stopper 33 may be properly set to regulate a direction of the loop formation part 16c in a process of developing the loop formation part 16c. For example, the wire passage part 33a formed at the stopper 33 may be directed in a direction that is inclined (e.g. orthogonal) with respect to a central line of the sheath 12 as illustrated in Fig. 12.

In the present embodiment, the loop formation part 16c forming a loop shape and the stopper 33 are together in contact with the resection target region, and a high-frequency current is carried. Thereby, the resection target region is resected like the first embodiment.

In the present embodiment, the sheath 12 may have the marking 30 (see Fig. 8) disclosed in the modification of the first embodiment.

Although the embodiments of the present invention have been described above in detail with reference to the drawings, the specific constitution is not limited to these embodiments, and also includes a change in design and so on without departing from the scope of the present invention.

For example, the second end 16h of the wire 16 disclosed in the third embodiment may extend up to a proximal side of the sheath 12 that freely moves forward and backward relative to the stopper 33 at the wire fixing part 33b and be coupled to the operation part 18. That is, the stopper 33 may have first and second passage parts in which the wire 16 of a U shape freely moves forward and backward.

In this case, another slider part for displacing the second end 16h is provided and manipulated for the operation part. Thereby, the second end 16h and the first end 16a of the wire 16 can be simultaneously displaced in a direction of the central line of the sheath 12, and a position of the folded part 16d relative to the distal end of the sheath 12 can be changed without developing the wire 16.

The components shown in each of the above embodiments and the modification can be properly configured by a combination thereof.

### [Industrial Applicability]

The present invention can be used in a medical device for performing the procedure of the EMRC.

### [Reference Signs List]

1, 1A, 1B Endoscopic tissue resection device (high-frequency treatment system)
2 Cap
3 Tube part
4 Endoscope coupler part
5 Contact part
6 Loop holder
7 Loop holding surface
10 Resection tool (high-frequency treatment tool)
11 Insertion part
12 Sheath
12A Sheath
14 Positioning member
14a Proximal end face
14b Through-hole part
16 Wire
16a First end
16b First wire part
16c Loop formation part
16d Folded part
16d1 Distal end face
16d2 First proximal portion
16d3 Second proximal portion
16e Second wire part
16f First connection part
16g Second connection part
16h Second end
17 Stopper
17a Distal end face
17b Wire fixing part
17c Wire passage part
18 Manipulator
19 Main body part
20 Shaft part
21 Finger hooking ring
22 Slider part
22A Another slider part
23 Finger hooking part
24 Connector
30 Marking
31 Inner sheath
32 Outer sheath
33 Stopper
50 Endoscope
51 Insertion body
52 Treatment tool channel
53 Image pickup unit
55 Manipulator
61 Stomach
62 Gullet
63 Gullet stomach joint region
64 Cardiac region
65 Mucosal layer, and so on
66 Squamocolumnar junction
67 Opening
68 Midriff
69 Gullet hiatus

## Claims

1. A high-frequency treatment tool used along with a cap that is attached to a distal end of an endoscope and the cap has a tubular inner circumferential surface, the high-frequency treatment tool comprising:
a tubular sheath having flexibility;
a first wire part disposed along a longitudinal axis of the sheath;
a loop formation part that is a wire continuous to a distal portion of the first wire part and enables elastic deformation to be bent in an annular shape when protruding from the distal portion of the sheath;
a second wire part disposed along the longitudinal axis of the sheath;
a folded part formed by folding a wire, which is configured to be projected from and retracted into a tip of the sheath and is continuous to a distal portion of the second wire part, in a U shape;
a connection part disposed between a distal portion of the loop formation part and a proximal portion of the folded part, configured to bend the proximal portion of the folded part in a direction apart from the distal portion of the second wire part, and connected to the distal portion of the loop formation part;
an operation part provided at a proximal portion of the first wire part and configured to enable manipulation such that the folded part, the loop formation part, and the connection part protrude from the tip of the sheath;
a stopper provided at a proximal portion of the second wire part and configured to have a diameter larger than an outer diameter of the second wire part; and
a positioning member having a contact surface set to come into contact with the stopper at a position at which an amount of protrusion of the loop formation part from the sheath along an axis of the loop formation part is shorter than a circumferential length of an inner circumferential surface at a distal end of the cap.

2. The high-frequency treatment tool according to claim 1, wherein the folded part and the connection part protrude from the tip of the sheath in a state in which the stopper is in contact with the contact surface of the positioning member.

3. The high-frequency treatment tool according to claim 1, further comprising a second connection part configured to bend the proximal portion of the folded part in a direction apart from the distal end of the first wire part between the distal portion of the second wire part and the proximal portion of the folded part and connect the distal portion of the second wire part and the proximal portion of the folded part.

4. The high-frequency treatment tool according to claim 3, wherein an interval between the wires of the folded part is smaller than an inner diameter of the sheath.

5. The high-frequency treatment tool according to claim 1, wherein:
the stopper has a fixing part to which a proximal end of the second wire part is fixed, and a passage part into which the first wire part is inserted to move forward and backward;
a distal end of the stopper and a proximal end of the positioning member come into contact with each other, and thereby the positioning member regulates movement of the second wire part; and
When a state in which the stopper and the positioning member are spaced from each other is changed to a state in which the stopper and the positioning member are in contact with each other, a part of the loop formation part is located in the sheath, and the folded part and the connection part protrude from the sheath.

6. The high-frequency treatment tool according to claim 1, wherein:
the sheath has an inner sheath in which the positioning member is fixed, and an outer sheath into which the inner sheath is inserted; and
in a state in which the positioning member regulate movement of the stopper, the outer sheath is capable of moving with respect to the inner sheath such that a distal end of the outer sheath is capable of being disposed close to the folded part.

7. The high-frequency treatment tool according to claim 1, wherein:
the positioning member has a first passage part into which the first wire part is inserted, and a second passage part into which the second wire part is inserted; and
the positioning member has conductivity and allows a high-frequency current to be carried.

8. The high-frequency treatment tool according to any one of claims 1 to 7, wherein the sheath has a marking at a position entering a visual field of the endoscope at a timing at which the folded part is located at a distal end portion of the cap.

9. A high-frequency treatment system comprising:
a cap attached to a distal end of an endoscope and having a tubular inner circumferential surface; and
a high-frequency treatment tool inserted into a channel of the endoscope, wherein
the high-frequency treatment tool includes:
a tubular sheath having flexibility;
a first wire part disposed along a longitudinal axis of the sheath;
a loop formation part that is a wire continuous to a distal portion of the first wire part and enables elastic deformation to be bent in an annular shape when protruding from the distal portion of the sheath;
a second wire part disposed along the longitudinal axis of the sheath;
a folded part formed by folding a wire, which is configured to be projected from and retracted into a tip of the sheath and is continuous to a distal portion of the second wire part, in a U shape;
a connection part disposed between a distal portion of the loop formation part and a proximal portion of the folded part, configured to bend the proximal portion of the folded part in a direction apart from the distal portion of the second wire part, and connected to the distal portion of the loop formation part;
an operation part provided at a proximal portion of the first wire part and configured to enable manipulation such that the folded part, the loop formation part, and the connection part protrude from the tip of the sheath;
a stopper provided at a proximal portion of the second wire part and configured to have a diameter larger than an outer diameter of the second wire part; and
a positioning member having a contact surface set to come into contact with the stopper at a position at which an amount of protrusion at the loop formation part from the sheath along an axis of the loop formation part is shorter than a circumferential length of an inner circumferential surface at a distal end of the cap; wherein
the cap has a loop holding surface that protrudes from the inner circumferential surface toward a central line of the cap and with which the folded part allows of contact.
